# EUROPEAN PATENT APPLICATION

(11) **EP 4 148 062 A1**
(43) Date of publication of application: **15.03.2023**
(21) Application number: 22461591.4
(22) Date of filing: 08.08.2022
(51) Int. Cl.: C07K 14/415, C12N 15/82

(54) **A PROMOTER ACTIVATED BY THE MYB47 AND MYB95 PROTEINS AND THE EXPRESSION SYSTEM CONTAINING IT**

(30) Priority: 06.08.2021 PL 43870321
(71) Applicant: UNIWERSYTET JAGIELLONSKI, 31-007 Kraków (PL); Instytut Chemii Bioorganicznej Polskiej Akademii Nauk, 61-704 Poznan (PL)
(72) Inventor: Sarkar, Shayan, 30-065 Kraków (PL); Kumar Basak, Arpan, Kraków (PL); Yamada, Kenji, 30-348 Kraków (PL); Bizan, Jakub, 63-300 Pleszew (PL); Bednarek, Pawe, 60-647 Poznan (PL); Czerniawski, Pawel, 61-245 Poznan (PL)
(74) Representative: Witek, Rafal

(57) **Abstract**

Disclosed is a promoter activated by the the MYB47 and MYB95 proteins and an expression system containing the same which may be used in plant biotechnology.

## Description

The subject of the invention is a promoter activated by the MYB47 and MYB95 proteins and an expression system containing it, which can be used in plant biotechnology.

Since humans selected and developed agricultural crops to exhibit high yield and good taste, agricultural crops became weaker than wild plants in the resistance against pests and diseases. It is a big problem how we can control plant pests to sustain sufficient food availability. Therefore, it is still desirable to increase the toolkit of available plant resistances.

Brassicaceae plant species and their close relative have developed unique endoplasmic reticulum (ER)-derived structures, namely ER bodies (also known as dilated cisternae or fusiform bodies) (Iversen, 1970; Ridge et al., 1999; Matsushima et al., 2003). ER bodies are subdomains of the ER that accumulate β-glucosidases (BGLUs), which possess an ER retention signal (Bonnett Jr and Newcomb, 1965; Matsushima et al., 2003).

Rosette leaves accumulate ER bodies in some larger epidermal cells (Nakazaki et al., 2019b; Nakazaki et al., 2019a). However, wounding of rosette leaves triggers the accumulation of ER bodies in the whole leaf epidermis via the perception of the wounding hormone j asmonate (JA) after leaf-wounding (Matsushima et al., 2002; Matsushima et al., 2003). Involvement of JA and wounding in ER-body formation further suggests an association of ER bodies with plant defense (Yamada et al., 2011; Nakano et al., 2014). The formation of JA-inducible ER bodies involves the induction of *TSA1* and *BGLU18* (Ogasawara et al., 2009; Stefanik et al., 2020).

MYB transcription factors comprise a large family of proteins that harbor a conserved DNA-binding domain and function in various biological processes (Martin and Paz-Ares, 1997; Jin and Martin, 1999; Stracke et al., 2001; Dubos et al., 2010). Most land plant MYB transcription factors are separated into subclades conserved across various species (Stracke et al., 2001; Millard et al., 2019; Jiang and Rao, 2020). However, some MYB transcription factors are unique to specific lineages and form lineage-specific subclades. In Arabidopsis, MYB47/95 clade is lineage-specific (Jiang and Rao, 2020).

The invention aims to provide new tools that can be used in plant biotechnology.

Surprisingly, the problem defined above is solved in the present invention.

The invention relates to a promoter activated the MYB47 and MYB95 proteins and an expression system containing it, as defined in the appended claims.

Inventors report that MYB47 and MYB95 regulate TSA1 and BGLU18 expression and JA-inducible ER-body formation in Arabidopsis. Both MYB47 and MYB95 recognized a DNA motif in the TSA1 promoter in vitro, and activated TSA1 gene promoter *in vivo.* Subject invention can be very useful for the agricultural industry, by increasing the "toolkit" for production of pest resistant plants.

Comparison of the promoter sequences of *TSA1* homologs in the Brassicaceae family revealed two conserved motifs, box1 (CACGTTTA) and box2 (GTTAGTT), in the region proximal to the start codon (Stefanik et al., 2020). Therefore, we examined whether MYB47 and MYB95 bind to box1 and box2 to regulate *TSA1* expression in Arabidopsis. Inventors found that both transcription factors bound to box2, but not to box1, *in vitro.* This binding specificity could be predicted since the sequence of box2 is the same as that of the MYB DNA-binding site (Myb 1 element) found in the promoters of defense related genes in tobacco and tomato (Pontier et al., 2001; Chakravarthy et al., 2003). In contrast to the *in vitro* assays, inventors found that both box1 and box2 elements are essential for the *TSA1* promoter activation by MYB47 and MYB95 *in vivo.* This finding indicates that box1 may have some role in the regulation of *TSA1* by MYB47 and MYB95. It suggests that MYB47 and MYB95 undergo post-translational modifications or associated with additional factor(s) to shift their DNA-binding specificity for the activation of the *TSA1* promoter in plant cells. Together, these results demonstrate that *TSA1* is a direct target of MYB47 and MYB95. BGLU18 is a component of the JA-inducible ER bodies (Ogasawara et al., 2009), implying that MYB47 and MYB95 directly regulate the *BGLU18* gene.

This description is further explained in the accompanying figures.

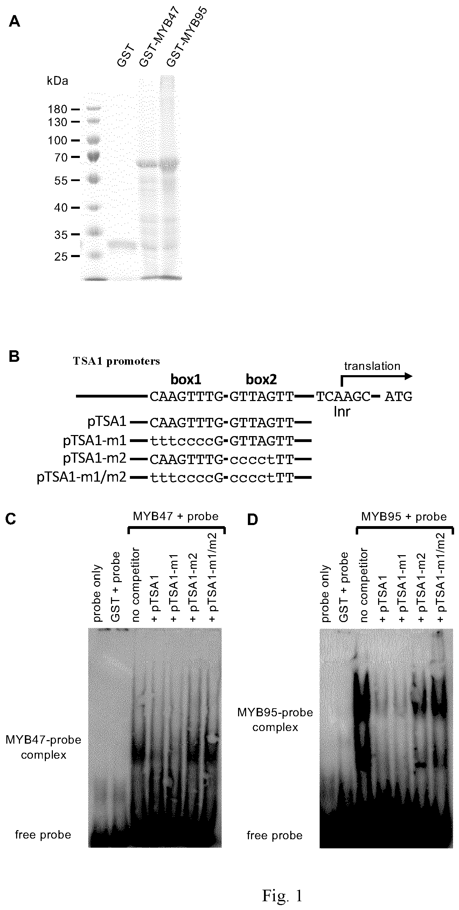

**Figure 1****.** MYB47 and MYB95 activate the *TSA1* promoter.
   **(A)** The affinity-purified GST-tagged fusion proteins were subjected to SDS-PAGE, followed by Coomassie staining.
   **(B)** Arabidopsis *TSA1* promoter harbors conserved sequences (box1 and box2), in addition to the initiator sequence (Inr). The sequences of box1 and box2 in the competitor oligonucleotides (pTSA1, pTSA1-m1, pTSA1-m2, and pTSA1-m1/m2) are shown. Lowercase letters denote mutated nucleotides.
   **(C)** Binding of MYB47 to the *TSA1* promoter *in vitro.* Partially purified GST-MYB47 fusion protein (Supplemental Figure S3) and a biotinylated-pTSA1 probe were used.
   **(D)** Binding of MYB95 to the *TSA1* promoter *in vitro.* Partially purified GST-MYB95 fusion protein (Supplemental Figure S3) and a biotinylated-pTSA1 probe were used.

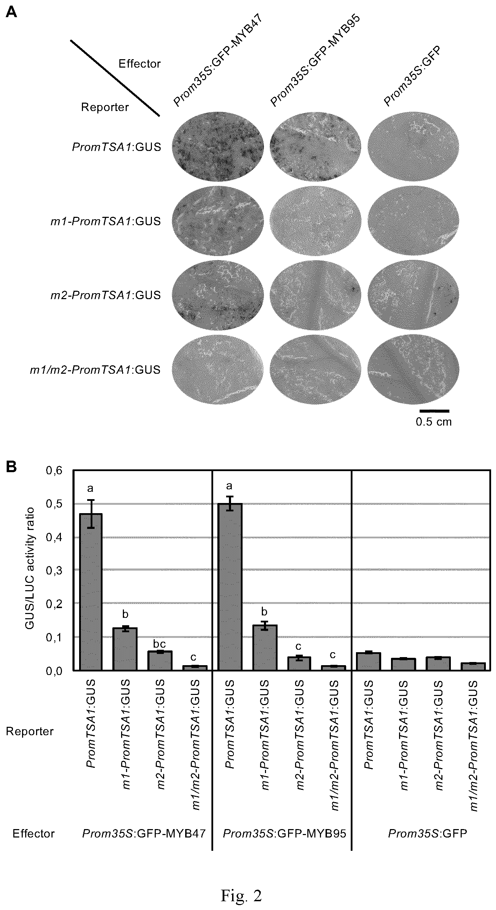

**Figure 2****.** Activation of the *TSA1* promoter by MYB47 and MYB95 in *Nicotiana benthamiana* leaves.
   **(A)** *Agrobacterium* harboring the effector construct was co-infiltrated into *N. benthamiana* leaves along with *Agrobacterium* harboring the reporter construct. Images show the GUS staining of *N. benthamiana* leaves. Scale bar = 0.5 cm.
   **(B)** Activation of the *TSA1* promoter by MYB47 and MYB95 *in planta.* The chart shows β-glucuronidase/luciferase (GUS/LUC) activity ratios measured 3 d after the agroinfiltration of *N. benthamiana* leaves with the effector, reporter and *Prom35S:LUC* constructs. Error bars represent SE (*n* = 6). Different lowercase letters indicate significant differences (*p* < 0.05; Tukey's test).

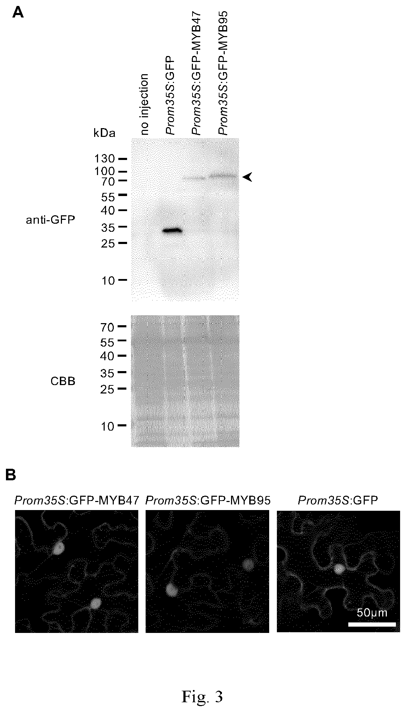

**Figure 3****.** Nuclear localization of MYB47 and MYB95.
   **(A)** Immunoblot analysis of GFP-MYB47 and GFP-MYB95 fusion proteins extracted from *Nicotiana benthamiana* leaves. Proteins were extracted from leaves infiltrated with *Agrobacterium* harboring the indicated constructs and subjected to immunoblot analysis with anti-GFP antibody. The arrowhead indicates the GFP-MYB47 or GFP-MYB95 fusion protein. Coomassie staining shows the amount of protein loaded in each lane.
   **(B)** Confocal laser scanning microscope image showing the GFP signal in *N. benthamiana* leaves agroinfiltrated with the indicated constructs. Scale bar = 50 µm.

### METHODS

### Plant Materials and Growth Conditions

Agroinfiltration experiments were conducted using *Nicotiana benthamiana* plants. *N. benthamiana* seeds were germinated in a greenhouse at 22°C under 16-h light/8-h dark photoperiod.

### Plasmid Construction

The Gateway binary vector *pGWB406* (a gift from T. Nakagawa) was used for constructing *Prom35S:GFP-MYB47* and *Prom35S: GFP MYB95.* The CDSs of *MYB47* and *MYB95* were transferred from the respective entry vectors into the *pGWB406* destination vector with LR clonase, thus generating the *pGWB406*/*MYB47* and *pGWB406*/*MYB95* vectors, respectively. Similarly, the Gateway binary vector *pGWB561* (a gift from T. Nakagawa) was used for constructing *Prom35S:tagRFP-MYC2, Prom35S:tagRFP-MYC3,* and *Prom35S:tagRFP-MYC4.* The CDSs of *MYC2, MYC3,* and *MYC4* were transferred from the respective entry vectors into the pGWB561 destination vector to generate *pGWB561*/*MYC2, pGWB561*/*MYC3,* and *pGWB561*/*MYC4* vectors, respectively.

The *TSA1* promoter sequence (~635 bp) was amplified from the genomic DNA of Arabidopsis ecotype Col-0 by PCR using pTSA1HindIIIF (AGACAAGCTTGTCTGTCCATGGATTGATAT) and pTSA1BamHIR (ACAGGGATCCAGCTTGAAGAAGCATCAC) primers. The PCR product was cloned into the pBI121 binary vector using the *HindIII* and *Bam*HI restriction endonucleases to generate the *pBI121*/*PromTSA1:GUS* construct. Two mutated versions of the *TSA1* promoter were generated by overlap extension PCR using pTSA1HindIIIF/mbox-pTSA1-R (mbox1-pTSA1-R, GAATGAAATCTTTCCCCAAGTTAGTTACAACGGTTT; mbox2-pTSA1-R, GAATGAAATCCACGTTTAACCCCTTTACAACGGTTT; mbox1mbox2-pTSA1-R, AAACCGTTGTAAAGGGGTTGGGGAAAGATTTCATTC) and pTSA1BamHIR/mbox-pTSA1-F (mbox1-pTSA1-F, GAATGAAATCTTTCCCCAAGTTAGTTACAACGGTTT; mbox2-pTSA1-F, GAATGAAATCCACGTTTAACCCCTTTACAACGGTTT; mbox1mbox 2-pTSA1-F, GAATGAAATCTTTCCCCAACCCCTTTACAACGGTTT) primer pairs. PCR products of the two reactions were pooled and subjected to another round of PCR using the pTSA1HindIIIF/pTSA1BamHIR primer pair. The mutated versions of the *TSA1* promoter were cloned into the pBI121 vector using the *Hind*III and *Bam*HI enzymes to generate *pBI121*/*m1-PromTSA1:GUS, pBI121*/*m2-PromTSA1:GUS,* and *pBI121*/*m1*/*m2PromTSA1:GUS* plasmids.

### Recombinant Protein Production

The recombinant GST-MYB47 and GST-MYB95 fusion proteins were produced in *E. coli* Rosetta 2 (DE3) cells (Merck) using the ZYP-5052 auto-induction medium, which contains terrific broth (TB) supplemented with 1 M MgSO₄, 50× 5052 (0.5% [w/v] glycerol, 0.05% [w/v] glucose, and 0.2% [w/v] lactose), 20× NPS (0.5 M (NH₄)₂SO₄, 1 M K₂HPO₄, and 1 M NaH₂PO₄) (Studier, 2005). Cells harboring the *pDEST15*/*MYB47* or *pDEST15*/*MYB95* plasmid were precultured in TB medium and then transferred to the induction medium. To produce GST-MYB47, cells were cultured at 30°C. To produce GST-MYB47, cells were first cultured at 37°C for 4-5 h (to obtain OD₆₀₀ >1.0) and then at 30°C overnight. Then, the cells were harvested and stored at -80°C.

### Agroinfiltration

*Agrobacterium tumefaciens* strains EHA105 and GV3101(pMP90RK) were precultured in lysogeny broth (LB) medium and then transferred to an induction broth medium (1 g/L NH₄Cl, 0.3 g/L MgSO₄·7H₂O, 0.15 g/L KCl, 10 mg/L CaCl₂, 2.5 mg/L FeSO₄·7H₂O, 2 mM sodium phosphate [pH 7.2], 20% [w/v] glucose, 20 mM MES-KOH buffer [pH 5.5], and 100 µM acetosyringone) supplemented with appropriate antibiotics (Winans et al., 1988). Agroinfiltration of *N. benthamiana* leaves was performed as described previously (Sarkar et al., 2021). Briefly, *Agrobacterium* cells were resuspended in the infiltration medium (0.5% [w/v] glucose, 10 mM MES-KOH [pH 5.5], 10 mM MgCl₂, and 200 µM acetosyringone) (OD₆₀₀ = 1.0). *Agrobacterium* cells carrying the p19 silencing suppressor (a gift from W. Strzalka) were resuspended in the infiltration medium (OD₆₀₀ = 0.5). Equal volumes of these *Agrobacterium* cell suspensions were mixed and infiltrated into the leaves of 4-5-week-old *N. benthamiana* plants.

### Recombinant GST-fusion Protein Purification

*E. coli* cells expressing the GST-MYB47 and GST-MYB95 fusion proteins were suspended in the extraction buffer (CelLytic B buffer [Merck] containing 0.1% [w/v] lysozyme, 0.01% [w/v] DNase I, and 1 mM PMSF), incubated on ice for 30 min, and lysed by sonication. Subsequently, the lysate was mixed with an equal volume of 1× phosphate buffered-saline (PBS) containing 0.1% (v/v) Triton X-100 at 4°C for 30 min, and the insoluble bacterial debris was removed by centrifugation. Then, DTT was added to the supernatant at a final concentration of 1 mM, and the soluble fractions were incubated with glutathione-sepharose 4B beads (GE Healthcare) at 4°C for 1.5 h with gentle rotation. The beads were transferred to a column and subsequently washed three times with 10× volume of PBS containing 0.1% (v/v) Triton X-100. The GST-MYB fusion proteins were eluted with a buffer containing 50 mM Tris-HCl (pH 8.0), 200 mM NaCl, and 20 µM glutathione. The eluant containing GST-MYB47 was subjected to ultrafiltration with Amicon Ultra Centrifugal Filter Unit Ultracel (3K MWCO, Merck) by exchanging the buffer with the dialysis buffer (1× PBS, 1 mM DTT, 5% [v/v] glycerol). The eluant containing GST-MYB95 was subjected to dialysis with the dialysis buffer at 4°C, and then concentrated by ultrafiltration.

### EMSA

The EMSAs were performed as described previously (Sarkar et al., 2021). Briefly, a 36-bp biotinylated double-stranded DNA probe and competitor DNAs were generated by annealing a 50-µM mixture of two complementary oligonucleotides (pTSA1-F, GAATGAAATCCACGTTTAAGTTAGTTACAACGGTTT; pTSA1-R, AAACCGTTGTAACTAACTTAAACGTGGATTTCATTC) in 10 mM Tris-HCl (pH 8.0), 50 mM NaCl, and 1 mM EDTA by heating to 95°C for 5 min and then slowly cooling to room temperature. The purified GST, GST-MYB47, or GST-MYB95 protein (500 ng) was incubated with the DNA probe in the binding buffer (10 mM Tris-HCl [pH7.5], 50 mM KCl, 1 mM DTT, 2.5% [v/v] glycerol, 5 mM MgCl₂, 0.1% [w/v] IGEPAL CA-630, and 0.05 µg/µL poly(dI-dC)) on ice. Competitor oligonucleotides (20-fold excess relative to the probe) were added to the binding reaction, and incubated at room temperature for 20 min. Following the addition of the pTSA1 probe at a final concentration of 5 µM, the mixture was further incubated at room temperature for 30 min, and the DNA-protein complexes were separated by PAGE on 5% (w/v) acrylamide/bis-acrylamide gel in 0.5× Tris/borate/EDTA (TBE) buffer. Samples were electrophoretically transferred from the gel on to a nylon membrane (Hybond-N⁺, Merck). The membrane was blocked with 3% (w/v) skim milk, and biotinylated DNA probe was detected with Avidin-HRP conjugate (Thermo-Fisher) by enhanced chemical luminescence (SuperSignal West Femto, Thermo-Fisher).

### Promoter-reporter transactivation assay

*Agrobacterium* cells harboring the effector construct (*Prom35S:GFP, Prom35S:GFP-MYB47,* or *Prom35S:GFP-MYB95*), reporter construct (*PromTSA1:GUS, m1-PromTSA1:GUS, m2-PromTSA1:GUS,* or *m1*/*m2-PromTSA1:GUS*)*,* and luciferase gene construct (*Prom35S:LUC*) were co-inoculated into the leaves of *N. benthamiana* plants. After 3 d, the inoculated leaves were harvested and homogenized in pre-cooled CCRL buffer (Promega). The samples were centrifuged twice for 15 min each at 10,000 rpm and 4°C. GUS activity was determined as described previously (Jefferson et al., 1987), with minor modifications. Aliquots of each supernatant (25 µL) were incubated with 75 µL of 10 mM 4-methylumbelliferyl-β-D-glucuronide, 0.1% (w/v) sodium lauryl sarcosine, and 10 mM β-mercaptoethanol in the CCRL buffer at 37°C for 0 min (control) or 30 min. The reactions were terminated with 180 µL of 0.2 M Na₂CO₃. The amount of the product (7-hydroxy-4-methylcoumarin) was measured using a microplate reader (Infinite 200, Tecan, Switzerland), at excitation and emission wavelengths of 355 and 460 nm, respectively. Aliquots of the above supernatants were also used to determine LUC activity using a LUC assay system kit (Promega). Protein concentration in each sample was measured with the Bradford method.

The GUS staining procedure was performed as described previously (Jefferson et al., 1987), with minor modifications. Briefly, leaves were cut into small pieces and vacuuminfiltrated in the GUS staining buffer (100 mM sodium phosphate [pH 7.0], 1 mM 5-bromo-4-chloro-3-indolyl-β-D-glucuronide, 0.5 mM potassium ferrocyanide, 0.5 mM potassium ferricyanide, and 10 mM EDTA) for 5 min. The leaf samples were incubated in the GUS staining buffer at 37°C for 16 h. The stained leaf tissues were then rinsed with ethanol and water to remove chlorophyll.

### Example 1

Because *TSA1* expression was downregulated in the *myb47 myb95* double knockout mutant, we examined the role of each MYB protein in the activation of the *TSA1* promoter using two approaches. First, we examined the binding of MYB47 and MYB95 to the *TSA1* promoter using electrophoretic mobility shift assays (EMSAs). Glutathione S-transferase (GST) fusions of MYB47 and MYB95 were separately expressed in *Escherichia coli* (Figure 1A), and the purified recombinant proteins were subjected to EMSA. The *TSA1* promoter region containing two *cis*-elements, box1 (CACGTTTA) and box2 (GTTAGTT), which are conserved among the Brassicaceae plant species (Stefanik et al., 2020), was used as the biotinylated DNA probe (hereafter referred to as pTSA1) (Figure 1B). Both MYB47 and MYB95 bound to the DNA probe in a box2-dependent manner; the addition of unlabeled pTSA1 or unlabeled pTSA1 containing a mutated box1 (pTSA1-m1), but not mutated box2 (pTSA1-m2), reduced the signal of probe-protein complexes (Figures 1C and 1D). Sequence of pTSA1 has been depicted as Seq. Id. No. 1.

### Example 2

Next, a promoter-reporter assay was conducted to examine the effect of MYB47 and MYB95 on the activity of the *TSA1* promoter. The β-glucuronidase (GUS) activity was induced in *Nicotiana benthamiana* leaves when co-inoculated with Agrobacterium harboring the *Prom35S:GFP-MYB47* (Seq. Id. No. 2) or *Prom35S: GFP MYB95* (Seq. Id. No. 3)construct and *Agrobacterium* harboring the *PromTSA1:GUS* construct (Seq. Id. No. 4). (Figure 2A and 2B). To determine the subcellular localization of MYB47 and MYB95 proteins, we transiently expressed the *GFP-MYB47* and *GFP-MYB95* fusions in the epidermal cells of *N. benthamiana* leaves (Figure 3A). Confocal microscopy analysis revealed strong GFP signal in the nucleus, in addition to weak signal in the cytosol (Figure 3B). These results indicate that MYB47 and MYB95 localize to the nucleus, consistent with their function as transcription factors. Consistent with the results of *in vitro* EMSAs, mutation in box2 (*m2-PromTSA1:GUS*) drastically reduced the *TSA1* promoter activity in promoter-reporter assays, despite the presence of MYB47 or MYB95 (Figure 2A and 2B). Unexpectedly, mutation in box1 (*m1-PromTSA1:GUS*) also reduced MYB47- and MYB95-dependent activation of the *TSA1* promoter (Figure 2A and 2B). Moreover, mutations in both box1 and box2 elements (*m1*/*m2-PromTSA1:GUS*) completely abolished the *TSA1* promoter activity (Figure 2A and 2B). These findings indicate that MYB47 and MYB95 activate the *TSA1* promoter in box1- and box2-dependent manner *in vivo.*

The following elements have been used in the described embodiments:
The coding sequence for the MYB47 and MYB95 protein cDNA is shown in Seq. Id. No. 5 and 6.

It encodes the MYB47 and MYB95 protein, the amino acid sequence of which is shown as Seq. Id. No. 7 and 8.

The coding sequence for the GFP-MYB47 and GFP-MYB95 fusion proteins are shown in Seq. Id. No. 9 and 10.

It encodes GFP-MYB47 and GFP-MYB95 fusions whose amino acid sequence is shown as Seq. Id. No. 11 and 12.

TSA1 promoter sequence shown in Seq. Id. No. 13.

### References

Bonnett Jr, H.T., and Newcomb, E.H. (1965). Polyribosomes and cisternal accumulations in root cells of radish. J. Cell Biol. 27, 423-432.
Chakravarthy, S., Tuori, R.P., D'Ascenzo, M.D., Fobert, P.R., Després, C., and Martin, G.B. (2003). The tomato transcription factor Pti4 regulates defense-related gene expression via GCC box and non-GCC box cis elements. Plant Cell 15, 3033-3050.
Dubos, C., Stracke, R., Grotewold, E., Weisshaar, B., Martin, C., and Lepiniec, L. (2010). MYB transcription factors in Arabidopsis. Trends Plant Sci. 15, 573-581.
Iversen, T.H. (1970). The morphology, occurrence, and distribution of dilated cisternae of the endoplasmic reticulum in tissues of plants of the Cruciferae. Protoplasma 71, 467-477.
Jefferson, R.A., Kavanagh, T.A., and Bevan, M.W. (1987). GUS fusions: β-glucuronidase as a sensitive and versatile gene fusion marker in higher plants. EMBO J. 6, 3901-3907.
Jiang, C.-K., and Rao, G.-Y. (2020). Insights into the diversification and evolution of R2R3-MYB transcription factors in plants. Plant Physiol. 183, 637-655.
Jin, H., and Martin, C. (1999). Multifunctionality and diversity within the plant MYB-gene family. Plant Mol. Biol. 41, 577-585.
Martin, C., and Paz-Ares, J. (1997). MYB transcription factors in plants. Trends Genet. 13, 67-73.
Matsushima, R., Hayashi, Y., Kondo, M., Shimada, T., Nishimura, M., and Hara-Nishimura, I. (2002). An endoplasmic reticulum-derived structure that is induced under stress conditions in Arabidopsis. Plant Physiol. 130, 1807-1814.
Matsushima, R., Hayashi, Y., Yamada, K., Shimada, T., Nishimura, M., and Hara-Nishimura, I. (2003). The ER body, a novel endoplasmic reticulum-derived structure in Arabidopsis. Plant Cell Physiol. 44, 661-666.
Millard, P.S., Kragelund, B.B., and Burow, M. (2019). R2R3 MYB transcription factors - Functions outside the DNA-binding domain. Trends Plant Sci. 24, 934-946.
Nakano, R.T., Yamada, K., Bednarek, P., Nishimura, M., and Hara-Nishimura, I. (2014). ER bodies in plants of the Brassicales order: Biogenesis and association with innate immunity. Front. Plant Sci. 5, 73.
Nakazaki, A., Yamada, K., Kunieda, T., Tamura, K., Hara-Nishimura, I., and Shimada, T. (2019a). Biogenesis of leaf endiolasmic reticulum body is regulated by both jasmonate-dependent and independent pathways. Plant Signal. Behav. 14, e1622982.
Nakazaki, A., Yamada, K., Kunieda, T., Sugiyama, R., Hirai, Y.M., Tamura, K., Hara-Nishimura, I., and Shimada, T. (2019b). Leaf endoplasmic reticulum bodies identified in Arabidopsis rosette leaves are involved in defense against herbivory. Plant Physiol. 179, 1515-1524.
Ogasawara, K., Yamada, K., Christeller, J.T., Kondo, M., Hatsugai, N., Hara-Nishimura, I., and Nishimura, M. (2009). Constitutive and inducible ER bodies of Arabidopsis thaliana accumulate distinct β-glucosidases. Plant Cell Physiol. 50, 480-488.
Pontier, D., Balagué, C., Bezombes-Marion, I., Tronchet, M., Deslandes, L., and Roby, D. (2001). Identification of a novel pathogen-responsive element in the promoter of the tobacco gene HSR203J, a molecular marker of the hypersensitive response. Plant J. 26, 495-507.
Ridge, R.W., Uozumi, Y., Plazinski, J., Hurley, U.A., and Williamson, R.E. (1999). Developmental transitions and dynamics of the cortical ER of Arabidopsis cells seen with green fluorescent protein. Plant Cell Physiol. 10, 1253-1261.
Sarkar, S., Stefanik, N., Kunieda, T., Hara-Nishimura, I., and Yamada, K. (2021). The Arabidopsis transcription factor NAI1 activates the NAI2 promoter by binding to the G-box motifs. Plant Signal. Behav. 16, 1846928.
Stefanik, N., Bizan, J., Wilkens, A., Tarnawska-Glatt, K., Goto-Yamada, S., Strzalka, K., Nishimura, M., Hara-Nishimura, I., and Yamada, K. (2020). NAI2 and TSA1 drive differentiation of constitutive and inducible ER body formation in Brassicaceae. Plant Cell Physiol. 61, 722-734.
Stracke, R., Werber, M., and Weisshaar, B. (2001). The R2R3-MYB gene family in Arabidopsis thaliana. Curr. Opin. Plant Biol. 4, 447-456.
Studier, F.W. (2005). Protein production by auto-induction in high-density shaking cultures. Protein Expr. Purif. 41, 207-234.
Winans, S.C., Kerstetter, R.A., and Nester, E.W. (1988). Transcriptional regulation of the virA and virG genes of Agrobacterium tumefaciens. J. Bacteriol. 170, 4047-4054.
Yamada, K., Hara-Nishimura, I., and Nishimura, M. (2011). Unique defense strategy by the endoplasmic reticulum body in plants. Plant Cell Physiol. 52, 2039-2049.

## Claims

1. A promoter activated by the MYB47 and MYB95 proteins containing the sequence shown in Seq. Id. No. 1.

2. The promoter according to claim 1 having the sequence shown in Seq. Id. No. 13.

3. An expression system comprising an effector cassette and a reporter cassette, **characterized in that** the effector cassette contains a gene encoding a MYB47 or MYB95 protein or a gene encoding a fusion protein comprising a MYB47 or MYB95 protein under the control of any known, preferably inducible, promoter, and the reporter cassette contains a gene encoding a reporter protein under the control of an promoter activated by a MYB47 or MYB95 protein, wherein the promoter activated by the MYB47 or MYB95 protein contains the sequence shown in Seq. Id. No. 1.

4. The expression system according to claim 3, **characterized in that** the promoter activated by a MYB47 or MYB95 protein has the sequence shown in Seq. Id. No. 13.

5. The expression system according to claim 3, **characterized in that** the gene encoding a MYB47 protein encodes a protein with the sequence shown in Seq. Id. No. 7.

6. The expression system according to claim 3, **characterized in that** the gene encoding a MYB95 protein encodes a protein with the sequence shown in Seq. Id. No. 8.

7. The expression system according to claim 3, **characterized in that** the gene encoding the MYB47 protein has the sequence shown in Seq. Id. No. 5.

8. The expression system according to claim 3, **characterized in that** the gene encoding the MYB95 protein has the sequence shown in Seq. Id. No. 6.

9. The expression system according to claim 3, **characterized in that** the gene encoding a fusion protein comprising MYB47 protein encodes a protein with the sequence shown as Seq. Id. No. 11.

10. The expression system according to claim 3, **characterized in that** the gene encoding a fusion protein comprising MYB95 protein encodes a protein with the sequence shown as Seq. Id. No. 12.

11. The expression system according to claim 3, wherein the gene encoding a fusion protein comprising MYB47 protein has the sequence shown in Seq. Id. No. 9.

12. The expression system according to claim 3, wherein the gene encoding a fusion protein comprising MYB95 protein has the sequence shown in Seq. Id. No. 10.

13. The expression system according to claim 3, wherein the effector cassette has the sequence shown in Seq. Id. No. 2 or 3.

14. The expression system according to claim 3, wherein the reporter cassette has the sequence shown in Seq. Id. No. 4.
